# EUROPEAN PATENT APPLICATION

(11) **EP 4 563 197 A1**
(43) Date of publication of application: **04.06.2025**
(21) Application number: 24215529.9
(22) Date of filing: 26.11.2024
(51) Int. Cl.: A61N 5/10

(54) **RADIATION TREATMENT PLAN OPTIMIZATION**

(30) Priority: 28.11.2023 US 202318520686
(71) Applicant: Siemens Healthineers International AG, 6312 Steinhausen (CH)
(72) Inventor: PELTOLA, Jarkko, 04300 Tuusula (FI); RUSANEN, Marko, 02940 Espoo (FI); TALLINEN, Tuomas, 02600 Espoo (FI); KESTI-HELIA, Anssi, 02940 Espoo (FI); VALENZUELA, Daniel, 0440 Helsinki (FI); FRIMAN, Anri, 02180 Espoo (FI); SABEL, Martin, 6332 Hagendorn (CH)
(74) Representative: Mathisen & Macara LLP

(57) **Abstract**

A control circuit can be configured to optimize a radiation treatment plan for a particular patient as a simultaneous function of using both volumetric modulated arc therapy and intensity-modulated radiation therapy techniques to provide an optimized radiation treatment plan. By one approach, the control circuit can be further configured to access information regarding at least one specific location along a treatment arc, in which case the aforementioned intensity-modulated radiation therapy can be correlated with the at least one specific location.

## Description

### Technical Field

These teachings relate generally to an energy-based treatment plan and more particularly to optimizing an energy-based treatment plan, for treating a patient's planning target volume with energy.

### Background

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In a modulated photon treatment planning workflow, the planner will typically decide whether to use an intensity-modulated radiation therapy (IMRT) plan (characterized by a set of fields with fixed gantry positions where each field modulates the incoming radiation using multi-leaf collimator leaves, jaws, and/or dose rate.) or a volumetric modulated arc therapy (VMAT) plan (characterized by a set of arc fields where the gantry rotates from a starting angle to a stopping angle while the irradiating beam is being modulated using multi-leaf collimator leaves, jaws, and/or dose rate.). The difference between the two treatments mainly comprises (a) setting up the fields and the number of them (in IMRT there are typically many fields (5 to 13) while in VMAT there are typically fewer fields (1 to 4), (b) the necessary treatment time (i.e., to accommodate a planned number of monitor units (Mus)), and (c) the shape of the resulting dose distribution.

IMRT field setup has the potential benefit where the user directly sets the direction of incoming radiation. The latter can permit better shaping of the resulting dose distribution. One potential downside of IMRT, however, is that the incoming fluence typically needs to be highly modulated (and hence necessitating up to hundreds of control points per direction) to produce good treatment. The latter, in turn, can lead to increased treatment time as compared to VMAT plans.

Also, simply moving from one IMRT field to another can require considerable time during a treatment session. In VMAT planning, controlling the direction for the incoming radiation is not as easy, but the treatment time is typically considerably faster since the dose is distributed from a sweep of gantry angles in a less modulated form (hence typically requiring fewer control points per 5 degree section of gantry rotation).

The applicant has identified a technical conundrum in the foregoing state of affairs. Given the foregoing options, it can be very difficult to achieve an arc treatment plan that is both temporally efficient with respect to treatment time but which can also accommodate a heightened concentration of dose when useful. In cases like breast treatments, for example, the lack of concentrated dosing in arc plans can potentially contribute to a less than optimum target dose coverage and/or a less steep dose fall-off at the target boundary. The latter concerns can, in turn, induce the planner to use IMRT fields and thereby accept the corresponding longer treatment time.

### Brief Description of the Drawings

The above needs and others are at least partially met through provision of the radiation treatment plan optimization described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a flow diagram as configured in accordance with various embodiments of these teachings;
FIG. 4 comprises a flow diagram as configured in accordance with various embodiments of these teachings;
FIG. 5 comprises a schematic view of an arc field as configured in accordance with various embodiments of these teachings; and
FIG. 6 comprises a screen shot as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### Detailed Description

Generally speaking, pursuant to these various embodiments, in one aspect the present invention provides a control circuit configured to optimize a radiation treatment plan for a particular patient as a simultaneous function of using both volumetric modulated arc therapy and intensity-modulated radiation therapy to provide an optimized radiation treatment plan. By one approach, the control circuit can be further configured to access information regarding at least one specific location along a treatment arc, in which case the aforementioned intensity-modulated radiation therapy can be correlated with the at least one specific location.

According to another aspect of the invention, the present invention further provides apparatus for administering a radiation treatment plan for a particular patient comprising a control circuit operably coupled to a memory and, optionally, a user interface and/or a network interface, an energy based treatment platform configured to deliver therapeutic energy to a patient, providing a particular radiation treatment platform having a source of radiation, in use, comprising an energy source, which energy source can be selectively moved via a gantry under the control of the control circuit, and one or more energy-shaping apparatus, wherein said control circuit accesses treatment arc information that identifies a treatment arc to be used with the particular radiation treatment platform while administering radiation to the particular patient, in use, while the source of radiation is moving according to the treatment arc accesses position information that identifies at least one specific location along the treatment arc where the source of radiation will halt to administer radiation to the particular patient, in use, while the source of radiation is halted; and optimizes a radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc. In some embodiments, the treatment arc comprises only a single direction of rotation.

According to another aspect of the invention, there is provided a method to facilitate optimizing a radiation treatment plan for a particular patient using a particular radiation treatment platform having a source of radiation, the method comprising, by a control circuit, accessing treatment arc information that identifies a treatment arc to be used with the particular radiation treatment platform while administering radiation to the particular patient while the source of radiation is moving according to the treatment arc; accessing position information that identifies at least one specific location along the treatment arc where the source of radiation will halt to administer radiation to the particular patient while the source of radiation is halted; optimizing a radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc. In some embodiments, the treatment arc comprises only a single direction of rotation.

By one approach, the optimized radiation treatment plan can provide both for administering volumetric modulated arc therapy radiation while the source of radiation is moving along the treatment arc and for administering intensity-modulated radiation therapy while the source of radiation is halted at the at least one specific location. If desired, the aforementioned administering of the intensity-modulated radiation therapy can comprise modulating radiation emitted by a source of radiation using at least one of a multi-leaf collimator, collimator jaws, and dose rate control.

By one approach, administering the volumetric modulated arc therapy comprises allowing a collimator to rotate while a corresponding radiation beam is on.

These teachings are both practical and flexible in practice, and will accommodate, for example, optimizing the radiation treatment plan by, at least in part, pre-populating treatment fields with initial radiation treatment platform control points, and then optimizing control point properties for the control points as a function, at least in part, of at least one cost function. In these regards, these teachings will further accommodate automatically altering the control points in response to at least one cost function. For example, altering the control points can comprise at least one of moving a control point, adding a control point, and deleting a control point.

By one approach, the aforementioned pre-populating of the treatment fields with initial radiation treatment platform control points can comprise assigning no more than one control point per degree of treatment arc for portions where volumetric modulated arc therapy is to be administered and assigning a plurality of control points to a single degree of the treatment arc for portions where intensity-modulated radiation therapy is to be administered.

By one approach, the aforementioned pre-populating of the treatment fields with initial radiation treatment platform control points can comprise populating the control points as a function of collimator rotation or any other selected moving axis. As an example, one could generate control points more densely in locations in the arc where a collimator rotates more than the gantry rotates.

In lieu of the foregoing, or in combination with any of the foregoing, the aforementioned optimizing of the radiation treatment plan for the particular patient as a simultaneous function of using both volumetric modulated arc therapy and intensity-modulated radiation therapy to provide the optimized radiation treatment plan can further comprise optimizing the radiation treatment plan for the particular patient as a simultaneous function of using both volumetric modulated arc therapy and intensity-modulated radiation therapy over a plurality of treatment sessions to provide the optimized radiation treatment plan.

By one approach, these teachings will facilitate optimizing a radiation treatment plan for a particular patient using a particular radiation treatment platform having a source of radiation by configuring a control circuit to access treatment arc information that identifies a treatment arc to be used with the particular radiation treatment platform while administering radiation to the particular patient while the source of radiation is moving according to the treatment arc, and then to access position information that identifies at least one specific location along the treatment arc where the source of radiation will halt to administer radiation to the particular patient while the source of radiation is halted. The control circuit can then optimize a radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc.

By one approach, the foregoing treatment arc comprises only a single direction of rotation.

By one approach, the aforementioned optimization of the radiation treatment plan can comprise optimizing the radiation treatment plan as a function of an arc field type that can permissibly contain temporary gantry rotation stops during traversal of the treatment arc while simultaneously modulating radiation emitted by the source of radiation. By one approach in such a case, optimizing the radiation treatment plan can comprise optimizing the radiation treatment plan as a function of a plurality of the arc field types. If desired, modulating the radiation emitted by the source of radiation can comprise modulating the radiation using at least one of a multi-leaf collimator, collimator jaws, and dose rate control.

By one approach, accessing the position information that identifies at least one specific location along the treatment arc where the source of radiation will halt to administer radiation to the particular patient while the source of radiation is halted can comprise providing a user with an opportunity to select the at least one specific location and receiving input from the user that selects the at least one specific location.

By one approach, accessing the position information that identifies at least one specific location along the treatment arc where the source of radiation will halt to administer radiation to the particular patient while the source of radiation is halted can comprise providing a user with an opportunity to select the at least one specific location and additionally select properties for the collimator angle at that specific location and receiving input from the user that selects the properties for the at least one specific location. Illustrative properties include, but are not limited to, the collimator angle and instructions for the pre-population of the control points on how to generate the intermediate collimator angles close to the specific location.

By one approach, accessing the position information that identifies at least one specific location along the treatment arc where the source of radiation will halt to administer radiation to the particular patient while the source of radiation is halted can comprise providing a user with an opportunity to select a weighting factor that weights (e.g., using a multiplier having any selected value from 0 to 1) how the pre-population of the control points and/or treatment plan optimization should change the control point properties at that location and receiving input from the user that selects the weight factor for the at least one specific location. Alternatively, a user can select a same weight factor to be used for all specific locations.

By one approach, optimizing the radiation treatment plan for the particular patient as a simultaneous function of both the treatment arc information and the position information can comprise accommodating a greater number of control points for when the source of radiation is halted at the at least one specific location along the treatment arc as compared to when the source of radiation is moving along the treatment arc.

By one approach, optimizing the radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc can comprise optimizing the radiation treatment plan for the particular patient as a further function of an aggregate effect of both treatment arc information and position information over a plurality of treatment sessions.

So configured, these teachings will support a new treatment planning method that combines aspects of both IMRT and VMAT planning. These teachings will accommodate a new arc field type that can contain temporary gantry rotation stops during the arc, while simultaneously modulating the radiation. These teachings will also accommodate a field-setup step and a treatment plan optimization algorithm that can optimize the new field. These teachings will also accommodate a control in the setup that can allow different scenarios for setting up the way regarding how a collimator rotates during the arc while simultaneously modulating the radiation. So configured, these teachings allow concentrated dosing where appropriate while avoiding the temporal penalty that typically accompanies such an approach. In particular, these teachings can provide for a radiation treatment that concentrates radiation at special points along a treatment arc, thus combining many of the best aspects of VMAT and IMRT planning while avoiding many of the less desirable aspects thereof.

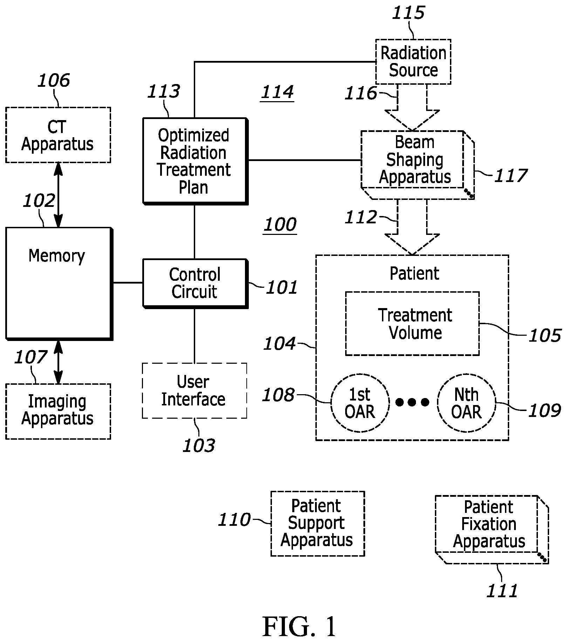

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to information such as optimization information for a particular patient, information regarding a particular radiation treatment platform, a neural network training corpus, and/or other input information as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

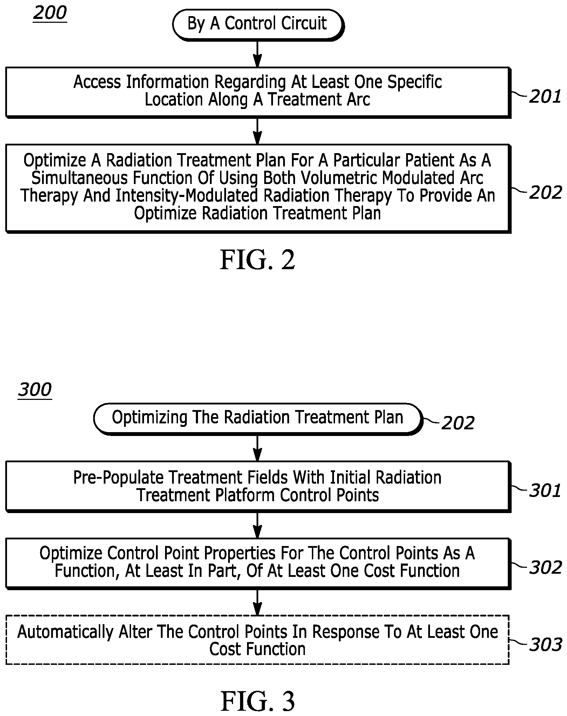

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for accessing information regarding at least one specific location along a treatment arc, and wherein the intensity-modulated radiation therapy is correlated with the at least one specific location. At block 202, the control circuit 101 can then optimize a radiation treatment plan for a particular patient as a simultaneous function of using both volumetric modulated arc therapy and intensity-modulated radiation therapy to provide an optimized radiation treatment plan. If desired, optimization of the plan can include a weighting factor that weights one or more of the properties of the plan between the volumetric modulated arc therapy sections and the intensity-modulated radiation therapy sections.

To be clear, the foregoing means what it says - optimization occurs as a simultaneous function of both such approaches to therapy. This will be understood to be different from, for example, first optimizing a plan as a function of volumetric modulated arc therapy and then subsequently optimizing the plan as a function of intensity-modulated radiation therapy.

FIG. 3 presents further elaboration regarding some approaches to such optimization.

At block 301, the control circuit 101 can pre-populate treatment fields with initial radiation treatment platform control points. By one approach, the foregoing can comprise assigning no more than one control point per degree of treatment arc for portions where volumetric modulated arc therapy is to be administered and assigning a plurality of control points to a single degree of the treatment arc for portions where intensity-modulated radiation therapy is to be administered. Here, if desired, the input weighting factor can be used to alter the population of control points. For example, if weight factor 0.5 refers to a default value leading to a default control-point population, then having a factor of 0.25 could mean that the number of control points in the halted gantry locations are scaled down by 50%. Vice-versa, a value of 1.0 could mean that the number of control points is scaled up from a default value by 100%. Also, the properties of the control points in those locations can be altered similarly (as one example, movement limits for leaves per control point). By one approach, the pre-population of the control points can take the amount of required collimator rotation and method into account. If the user has selected different collimator angles for two adjacent special locations (examples including, but not limited to, a start position of the arc field, a start position of an avoidance sector, an end position of an avoidance sector, an end position of the arc field, and/or a position of the halted gantry) and an instruction method regarding how to handle collimator rotation during gantry movement between adjacent locations, the pre-population of control points can produce different amounts of control points per gantry angle as a function of collimator angle rotation during the locations. Pre-population can also result in rotating the collimator between such locations only at the start/end of the special locations while the gantry is halted and the beam is turned off. This method avoids rotating a collimator during beam-on windows while still allowing different collimator rotation angles to be used in different special locations. Pre-population can also optimize the rotation paths between special locations based on a separate trajectory optimization algorithm. Pre-population can also optimize the rotation paths between special locations and in the locations themselves based on a separate trajectory optimization algorithm.

At block 302, the control circuit 101 can then optimize control point properties for the control points as a function, at least in part, of the aforementioned one or more cost functions. (Those skilled in the art understand that optimizing can comprise evaluating a cost function for multiple potential solutions, and finding a final solution with a small (ideally, but not necessarily, the smallest) cost function value. The cost function can be generated, for example, by splitting clinical goals (such as prescribed radiation doses) into a clinical metric (such as a target dose coverage or dose-volume goals for organs-at-risk) and a corresponding threshold value (i.e., an optimization objective). Each clinical goal can then be converted into a term (such as a quadratic term), with each term defined as the difference (such as the quadratic difference) between the value of a metric calculated for the candidate solution (related to a corresponding clinical goal metric) and a threshold value (related to the objective value of the corresponding clinical goal). Before a corresponding summation, each term can be multiplied by an individual weight determined based on relative priorities of the different goals. Various cost function approaches are known in the art. As the present teachings are not overly dependent upon the selection of any particular approach, no further elaboration is provided here for the sake of brevity.)

By one optional approach, and as shown at block 303, these teachings will accommodate configuring the control circuit to automatically alter one or more control points in response to at least one cost function. The latter may comprise, as desired, any one or more of moving one or more control points, adding one or more control points, and/or deleting one or more control points.

Further details that comport with these teachings will now be presented. It will be understood that the specific details of these examples are intended to serve an illustrative purpose and are not intended to suggest any particular limitations with respect to these teachings.

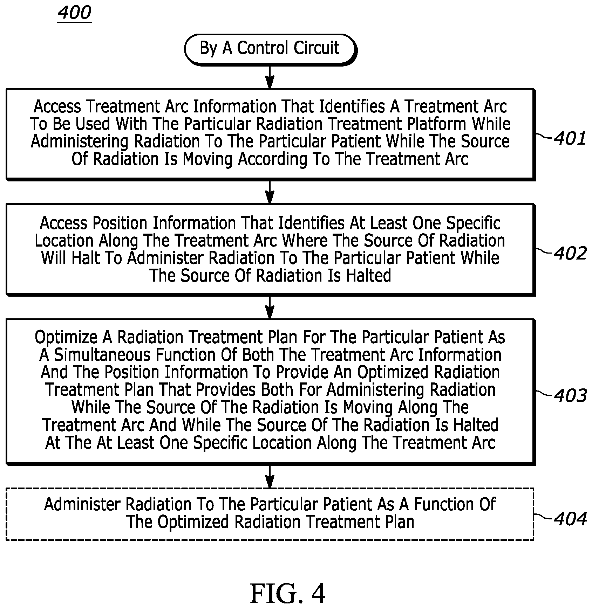

FIG. 4 presents a process 400 where, at block 401, the control circuit 101 accesses treatment arc information that identifies a treatment arc to be used with the particular radiation treatment platform while administering radiation to a particular patient while the source of radiation is moving according to the treatment arc. For the sake of a simple example, it will be presumed here that this treatment arc comprises only a single direction of rotation (for example, only a clockwise direction of rotation or only a counter-clockwise direction of rotation). The arc field, if desired, can include one or more sectors where the beam is to be turned off (sometimes referred to herein as "avoidance sectors").

At block 402, the control circuit 101 access position information that identifies at least one specific location along the treatment arc where the source of radiation will halt to administer radiation to the particular patient while the source of radiation is halted. The latter can include, if desired, providing a user with an opportunity (via, for example, the aforementioned user interface 103) to select the at least one specific location (by, for example, clicking on a displayed cursor or other selection icon or tool) and then receiving corresponding input from the user that selects the at least one specific location. (These teachings will optionally accommodate permitting a user to make other related selections as well if desired. For example, a user may be permitted to select a specific collimator angle and/or a specific collimator jaw limitation for a selected specific location. As another example, the user may be provided with the opportunity and mechanism to select a method specifying how a collimator angle will change between specified locations.)

At block 403, the control circuit 101 can then optimize a radiation treatment plan for the particular patient as a simultaneous function of both the aforementioned treatment arc information and the aforementioned position information to provide a resultant optimized radiation treatment plan that provides both for administering radiation while the source of radiation is moving along the treatment arc and also while the source of radiation is halted at the at least one specific location along the treatment arc.

By one approach, the foregoing optimization of the radiation treatment plan for the particular patient as a simultaneous function of both the treatment arc information and the position information can include accommodating a greater number of control points for when the source of radiation is halted at the at least one specific location along the treatment arc as compared to when the source of radiation is moving along the treatment arc.

By another approach, in lieu of the foregoing or in combination therewith, the foregoing optimization of the radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc can include optimizing the radiation treatment plan for the particular patient as a further function of an aggregate effect of both treatment arc information and position information over a plurality of treatment sessions.

By one approach, the aforementioned optimization of the radiation treatment plan can comprise optimizing the radiation treatment plan as a function of an arc field type that can permissibly contain temporary gantry rotation stops during traversal of the treatment arc while simultaneously modulating radiation emitted by the source of radiation. In such a case, optimizing the radiation treatment plan can comprise, at least in part, optimizing the radiation treatment plan as a function of a plurality of different arc field types. By one approach, the aforementioned modulation of the radiation emitted by the source of radiation can comprise modulating the radiation using at least one of a multi-leaf collimator, collimator jaws, and/or dose rate control and any combination thereof.

At optional block 404, radiation can be administered to the particular patient as a function of the resultant optimized radiation treatment plan 113 using, for example, the above-described radiation treatment platform 114.

It will be appreciated that these teachings provide for a new radiation treatment planning method that combines aspects of both IMRT and VMAT planning. These teachings in particular will accommodate a new arc field type that can contain temporary gantry rotation stops during a traversal of the arc, while simultaneously modulating the corresponding radiation. These teachings will further accommodate a field-setup step and a treatment plan optimization algorithm that can optimize this new field.

These teachings will also permit a user to define a new photon treatment plan type. This plan type can consist of one or more new arc treatment fields that enable the gantry to rotate and stop multiple times sequentially while leaf positions, jaw positions, and dose rate serve to modulate the radiation. In particular, during gantry rotation the collimator can be allowed to rotate while the radiation beam is on while during gantry stop points, the leaves, jaws, and dose rate can be modulating the radiation. By one approach, the radiation beam can be turned off during parts of the field.

A treatment plan consisting of a number of these new arc fields can be optimized simultaneously. The delivery of each field can be continuous, thus combining at least many of the best parts of IMRT and VMAT characteristics while also avoiding or at least mitigating many of the problems presented by prior art approaches. For example, when considering treatment of a breast tumor, these teachings allow user to set up an arc and then select two tangential directions (such as, for example, traditional IMRT treatment field gantry angles) from that arc. The corresponding treatment can concentrate radiation at those special points while essentially employing VMAT-style treatment elsewhere on the arc.

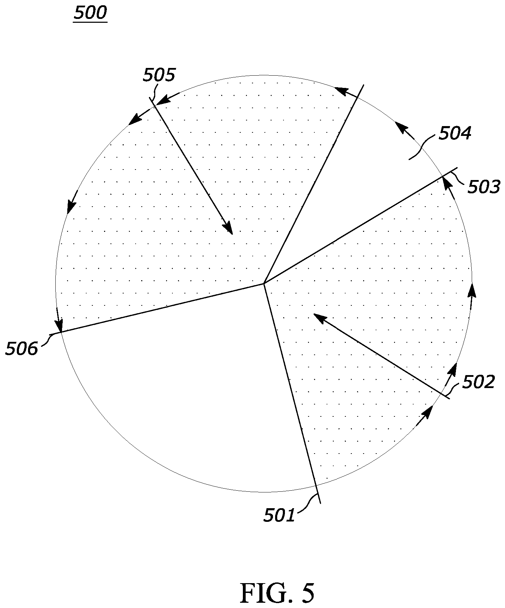

FIG. 5 presents a schematic representation of an illustrative example of a single continuous arc field 500 in accord with these teachings.

The start of the arc field is denoted by reference numeral 501. Control point properties here are as follows: gantry = 170 deg, collimator = 45 deg, and field max size = 10 cm x 10 cm). The radiation source applies radiation to the patient as the source moves counter-clockwise on the arc until the radiation source reaches a first gantry stopping point that is denoted by reference numeral 502. Control point properties here are: gantry = 130 deg, collimator = 25 deg, and field max size = 15 cm x 15 cm.

After stopping and applying radiation per the optimized plan, the radiation source again moves counter-clockwise will "on" until the radiation source reaches a beam-off point (denoted by reference numeral 503) that begins a beam-off sector 504. Beginning at this point 503, the radiation source continues rotating on the gantry but the radiation source is "off' and emits no radiation. Control point properties here are: gantry = 70 deg, collimator = 45 deg, and field max size = 10 cm x 10 cm). Upon reaching the conclusion of the beam-off sector, the radiation source again emits radiation while continuing to traverse the arc. At the end of the beam-off sector 504, control point properties are: gantry = 40 deg, collimator = 55 deg, and field max size = 15 cm x 15 cm.

At the point denoted by reference numeral 505, the radiation source reaches a second gantry stopping point. Control point properties here are gantry = -30 deg, collimator = 55 deg, and field max size = 15 cm x 15 cm. When planned treatment at this second stopping point 505 concludes, the radiation source again begins moving along the arc while also continuing to irradiate the target(s).

Finally, at the point denoted by reference numeral 506, the radiation source reaches the end of the arc field and the beam is switched "off." Control point properties here are: gantry = -100 deg, collimator = 45 deg, and field max size = 10 cm x 10 cm.

An illustrative example of a planning flow in a treatment planning can include the following steps:
define an iso-center for a new patient image;
define fields by adding new arc fields and select (only a) few gantry directions from the arc for concentrated gantry stop directions using patient image cross-section as a visual guide;
fine-tune collimator rotations and maximum field sizes for each stop direction and the underlying arc using a beam's-eye-view as a visual guide;
set additional optimizer parameters for the fields and their parts for the optimizer to know how to set and limit the control points and their properties, that it creates;
optimize a radiation treatment plan using corresponding optimization objectives;
calculate the corresponding dose;
send the optimized plan to the radiation treatment platform which follows the optimized control point sequence continuously for each field by using leaves, jaws, and dose rate to modulate the incoming radiation, stopping the gantry during the arc when specified by the plan, and rotating the collimator during gantry rotations when specified by the plan.

Some illustrative examples of field set up will now be provided.

Following a normal arc field setup workflow, and following isocenter placement and the defining of a set of arcs (defined, for example, by the starting and ending gantry angles for each field), a user will be able to select 1 through N gantry angles (these being the intended static points) from each arc for introducing concentrated radiation directions (thereby mimicking IMRT field directions). Additionally, if desired, the user can also define arc angle ranges from each arc where the beam should be off (so called stop-beam or avoidance sectors).

The aforementioned selected points on the arcs can be further set to have different collimator angles or maximum field size openings for the collimation jaws (the jaw positions can be optimized to move within the maximum field size during the optimization phase). When applying different collimator angles (i.e., different from each other and/or from the arc's start and end positions), the user can be provided with an option to configure the optimization algorithm to create dynamic collimator rotation in any of a variety of different ways while the gantry is moving. Some illustrative examples in these regards include:
linearly interpolated rotation between the selected ports (and start/end positions of the arc) while the beam is on;
optimized rotation between the selected ports (and start/end positions of the arc, or start/end positions of the arc sectors where the beam is off) while the beam is on; or
no rotation while the beam is on, but rotation is allowed when the beam is off when entering or exiting a static point (also sometimes referred to herein as a static port).

Since the field setup has multiple options, these teachings will accommodate providing the user with the ability to graphically and/or textually define the geometry of the arcs (including such things as start/end angles, collimator rotation, maximum field size, and so forth), static port placement (for example, by selecting gantry angles and setting collimator rotation and maximum field size), and set parameters to control what happens between ports (for example, specifying collimator rotation mode between specific ports) and during the ports (for example, via importance/weight factors or other limitations or characterizations). If desired, the user interface 103 can be connected to a beam-eye-view visualization of the relevant geometry for easier alignment of the field sizes and rotations in the ports.

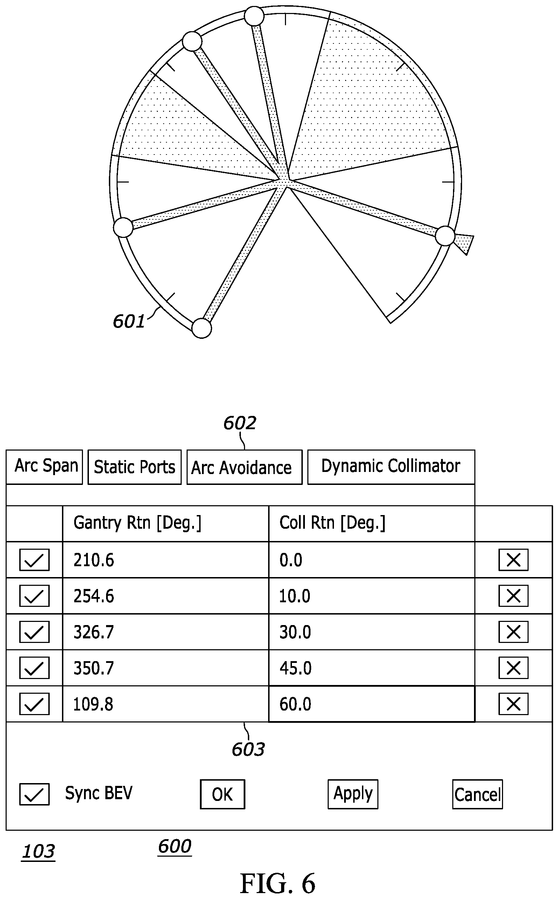

FIG. 6 provides an illustrative example of a window 600 that can be provided in the user interface 103 (either as a full-screen window or as an overlaying window). Such a window 600 can present, for example, a schematic representation of an arc field 601 including start points, end points, stop points, and regions where the radiation source is on or off when the source is moving along the arc path. A series of tabs can provide ready access to corresponding tables 603 that provide alphanumeric entry and display fields pertaining to such things as arc spans, static port locations and control point context, arc avoidance, and dynamic collimator settings.

If desired, these teachings will accommodate automating at least initial static field placement using an algorithm, a heuristic rule, a template, or some other enabling approach of choice. One such algorithm could be, for example, a static field placement based on target and healthy tissue projections on the multi-leaf collimator plane.

As described above, after the field setup pursuant to these teachings, one or more static ports can be a part of some or all of the treatment fields. Also pursuant to these teachings, the foregoing can be optimized simultaneously and efficiently with the arc parts.

By one approach, a corresponding optimization algorithm can include first populating the fields with initial machine control points and then optimizing the control point properties (for example, leaf positions, jaw positions, and meter set weights) using a standard cost function for the treatment plan. These teachings will also accommodate altering the initial control point positions and/or adding or removing control points during the optimization process based on the cost function and/or a heuristic rule of choice.

By one illustrative approach, an initial control point population can set one control point per 1 or 2 degrees of gantry rotation in the parts where the gantry is rotating while the beam is on. For static gantry ports, however, the number of control points can vary per each static port but nevertheless while likely being greater in number than where the beam is on while moving still being likely less than hundreds of control points (the latter being not untypical for a standard IMRT field). The latter, in turn, can help keep the amount of monitor units low and treatment time uncompromised.

The default number of control points at static gantry ports could be, for example, 50. If the user selects the collimator to rotate during gantry rotation, the rotation can be interpolated directly between set angles in static ports and the start/end points of the arc. The collimator rotation can also be optimized using a separate algorithm that can create collimator rotation patterns according to requested parameters such as structure information (target, oar), importance based on the objectives, and/or machine limitations. The rotation can also be limited to not cause the gantry rotation to slow down if desired. The rotation can also be used as an input to generate more control points along the path of gantry rotation.

Optimization of control point properties could be done using any optimization method that can minimize a cost function by altering the control point properties and that understands the machine limitations (for example, so-called Direct Aperture Optimization (DAO)). The optimization cost function could contain standard DVH objectives (such as upper/lower and exact dose volume points/lines per structure), generalized equivalent uniform dose (gEUD) objectives, total monitor units, count objectives, and/or aperture shape objectives, just to mention a few.

These teachings will accommodate an optimization approach having an additional cost function term/weighting term that can differentiate between the incoming dose/fluence coming from the parts where the gantry is rotating and the parts where the gantry is stationary/static. In some treatment cases, the user might want to emphasize the importance of the static parts over the arc parts, and in some cases the opposite. This can be implemented either as a relative weight parameter or as a cost term for different parts.

A control variable can act either in fluence space or dose space. For fluence space, the control mechanism can use any metric that affects the fluence from the different parts (such as, the amount of monitor units in the parts, complexity of the fluence modulation represented by the number of control points, time spent on the parts, an amount of collimator leaf travel or the form of leaf movement, and so forth). At least for some application settings, a simplest method may be to initialize the optimization process with control points where the number of static gantry part control points and their property limits are scaled with the given weight parameter. Another way to control this is via dose distribution by adjusting any dose-based cost function term to alter the way how different parts participate in the optimization of the dose distribution. For example, a weight parameter could represent that a maximum/exactly/minimum (as selected) of 80% of the chosen metric is expected to be provided by the arc part control points, and 20% is provided by the static part's control points. As another example, the weights could be textually represented by such meaningful expressions as "default," "static part dominant," "arc part dominant," and so forth. If desired, the optimization algorithm could act to limit the control point properties in different parts (such as parts where the gantry is moving as versus where the gantry is static) differently.

The effective combination of IMRT and VMAT treatment techniques into a single treatment field offers the possibility of improving arc treatment plan quality via additional degrees of freedom. The ability to concentrate the dose from some arc parts can allow better dose fall-off control and encourage better target dose conformality with fewer arc fields being needed. The new field type, optimized with, for example, the above-described DAO-based optimization approach, allows a plan to effect IMRT style treatments with a considerably reduced monitor unit count while also being able to improve the dose distribution shape using the arc parts.

Using the above-described dynamic collimator rotation can help reduce the dosing of organs-at-risk by choosing best angles at each control point during gantry rotation. At the same time, these teachings permit reducing the number of arc fields as compared to a normal arc field plan that might produce similar plan quality.

Those skilled in the art will recognize that a wide variety of modifications, alterations, and combinations can be made with respect to the above described embodiments without departing from the scope of the invention. As but one example in these regards, these teachings will accommodate adding additional planning capabilities to the utilized plan optimization algorithm. In this way, and as one example, these teachings will accommodate an automatic skin-flash feature that could allow the user to define an extension of the target structure volume so that movement during treatment is taken into account in the resulting plan. It will therefore be understood that such modifications, alterations, and combinations are to be viewed as being within the ambit of the inventive concept.

## Claims

1. Apparatus (100) for administering a radiation treatment plan for a particular patient (104) comprising a control circuit (101) operably coupled to a memory (102), the control circuit being operably coupled to an energy based treatment platform (114) configured to deliver therapeutic energy (112) to a patient (104), in use, said treatment platform comprising an energy source (115), which energy source (115) can be selectively moved via a gantry under the control of the control circuit (101), and one or more energy-shaping apparatus (117), wherein:
said control circuit (101) is configured to:
access treatment arc information (401) that identifies a treatment arc to be used with the particular radiation treatment platform while administering radiation to the particular patient (104), in use, while the source of radiation (115) is moving according to the treatment arc;
access position information (201, 402) that identifies at least one specific location along the treatment arc where the source of radiation (115) will halt to administer radiation to the particular patient (104), in use, while the source of radiation is halted; and
optimize a radiation treatment plan (403) for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc, optionally the treatment arc comprises only a single direction of rotation.

2. The apparatus of claim 1 wherein optimizing the radiation treatment plan comprises optimizing the radiation treatment plan as a function of an arc field type that can permissibly contain temporary gantry rotation stops during traversal of the treatment arc while simultaneously modulating radiation emitted by the source of radiation, optionally optimizing the radiation treatment plan comprises optimizing the radiation treatment plan as a function of a plurality of the arc field types,wherein optionally, the modulating of the radiation emitted by the source of radiation comprises modulating the radiation using at least one of a multi-leaf collimator, collimator jaws, and dose rate control.

3. The apparatus of claim 1 or 2, wherein accessing the position information that identifies at least one specific location along the treatment arc where the source of radiation will halt to administer radiation to the particular patient, in use, while the source of radiation is halted comprises:
providing a user with an opportunity to select the at least one specific location;
receiving input from the user that selects the at least one specific location.

4. The apparatus of claim 1, 2 or 3, wherein optimizing the radiation treatment plan for the particular patient as a simultaneous function of both the treatment arc information and the position information further comprises accommodating a greater number of control points for when the source of radiation is halted at the at least one specific location along the treatment arc as compared to when the source of radiation is moving along the treatment arc.

5. The apparatus of any one of the preceding claims, wherein said control circuit optimizes the radiation treatment plan for the particular patient as a further function of an aggregate effect of both treatment arc information and position information from a plurality of treatment sessions.

6. A control circuit configured to
optimize a radiation treatment plan for a particular patient as a simultaneous function of using both volumetric modulated arc therapy and intensity-modulated radiation therapy to provide an optimized radiation treatment plan (202).

7. The control circuit of of claim 6 further configured to
access information regarding at least one specific location along a treatment arc; and wherein the intensity-modulated radiation therapy is correlated with the at least one specific location.

8. A method of using the control circuit of claim 6 or 7, wherein the optimized radiation treatment plan provides both for administering volumetric modulated arc therapy radiation while the source of radiation is moving along the treatment arc and for administering intensity-modulated radiation therapy while the source of radiation is halted at the at least one specific location, in use, wherein optionally administering the intensity-modulated radiation therapy comprises modulating radiation emitted by a source of radiation using at least one of a multi-leaf collimator, collimator jaws, and dose rate control, wherein optionally, administering the volumetric modulated arc therapy comprises allowing a collimator to rotate while a corresponding radiation beam is on.

9. The method of claim 8, wherein optimizing the radiation treatment plan (202) comprises:
pre-populating treatment fields with initial radiation treatment platform control points (301);
optimizing control point properties for the control points as a function, at least in part, of at least one cost function (302).

10. The method of claim 9 further comprising:
automatically altering the control points in response to at least one cost function (303).

11. The method of claim 9 or 10 wherein altering the control points comprises at least one of moving a control point, adding a control point, and deleting a control point.

12. The method of any one of claims 9-11, wherein pre-populating treatment fields with initial radiation treatment platform control points comprises assigning no more than one control point per degree of treatment arc for portions where volumetric modulated arc therapy is to be administered and assigning a plurality of control points to a single degree of the treatment arc for portions where intensity-modulated radiation therapy is to be administered.

13. The method of any one of claims 9 -12, wherein optimizing the radiation treatment plan for the particular patient as a simultaneous function of using both volumetric modulated arc therapy and intensity-modulated radiation therapy to provide the optimized radiation treatment plan further comprises optimizing the radiation treatment plan for the particular patient as a simultaneous function of using both volumetric modulated arc therapy and intensity-modulated radiation therapy over a plurality of treatment sessions to provide the optimized radiation treatment plan.

14. A method to facilitate optimizing a radiation treatment plan for a particular patient using a particular radiation treatment platform having a source of radiation, the method comprising:
by a control circuit (101):
accessing treatment arc information (201, 401) that identifies a treatment arc to be used with the particular radiation treatment platform while administering radiation to the particular patient while the source of radiation is moving according to the treatment arc;
accessing position information (402) that identifies at least one specific location along the treatment arc where the source of radiation will halt to administer radiation to the particular patient while the source of radiation is halted;
optimizing a radiation treatment plan (202, 403) for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc,
wherein, optionally, the treatment arc comprises only a single direction of rotation.

15. The method of claim 14, wherein optimizing the radiation treatment plan comprises optimizing the radiation treatment plan as a function of an arc field type that can permissibly contain temporary gantry rotation stops during traversal of the treatment arc while simultaneously modulating radiation emitted by the source of radiation, optionally,
optimizing the radiation treatment plan comprises optimizing the radiation treatment plan as a function of a plurality of the arc field types, optionally,
modulating the radiation emitted by the source of radiation comprises modulating the radiation using at least one of a multi-leaf collimator, collimator jaws, and dose rate control, optionally, accessing the position information that identifies at least one specific location along the treatment arc where the source of radiation will halt to administer radiation to the particular patient while the source of radiation is halted comprises providing a user with an opportunity to select the at least one specific location and receiving input from the user that selects the at least one specific location, optionally,
optimizing the radiation treatment plan for the particular patient as a simultaneous function of both the treatment arc information and the position information further comprises accommodating a greater number of control points for when the source of radiation is halted at the at least one specific location along the treatment arc as compared to when the source of radiation is moving along the treatment arc, optionally,
optimizing the radiation treatment plan for the particular patient as a simultaneous function of both the treatment arc information and the position information to provide an optimized radiation treatment plan that provides both for administering radiation while the source of radiation is moving along the treatment arc and while the source of radiation is halted at the at least one specific location along the treatment arc further comprises optimizing the radiation treatment plan for the particular patient as a further function of an aggregate effect of both treatment arc information and position information over a plurality of treatment sessions.
